# EUROPEAN PATENT SPECIFICATION

(11) **EP 2 623 161 B1**
(45) Date of publication and mention of the grant of the patent: **28.10.2015**
(21) Application number: 13153765.6
(22) Date of filing: 01.02.2013
(51) Int. Cl.: A62C 35/60, A62C 37/11, A62C 3/00, A62C 35/68, A62C 37/14, A62C 35/62

(54) **Flexible dry sprinklers**
Flexible Trockensprinkler
Gicleurs secs flexibles

(30) Priority: 03.02.2012 US 201261594972 P
(43) Date of publication of application: 07.08.2013
(73) Proprietor: The Reliable Automatic Sprinkler Co., Inc., Liberty ,SC 29657 (US)
(72) Inventor: Multer, Thomas, Liberty, SC South Carolina 29657 (US)
(74) Representative: Curley, Donnacha John

(56) References cited:
- WO-A1-2012/166636
- WO-A1-2012/166644
- US-A- 1 405 411
- US-A1- 2003 075 343
- US-B1- 6 488 097

## Description

### BACKGROUND

Dry sprinklers are used in areas that are exposed to freezing conditions, such as in freezers or walkways that may experience freezing conditions. In some dry-pipe systems supply conduits run in a space where the fluid in the supply conduit is not subject to freezing. A dry-sprinkler is attached to the supply conduit and extends into a space where the fluid would otherwise be subject to freezing.

The typical construction of a dry sprinkler comprises a sprinkler head, a tube, a pipe connector at the inlet end of the tube (for connecting the inlet end to the pipe network of the fire suppression system), a plug seal at the inlet end to prevent water from entering the tube, and an actuating mechanism to maintain the plug seal at the inlet end. Typically, the sprinkler head is attached to the end of the tube opposite to the inlet end of the tube. Also, the tube section is conventionally vented to the atmosphere to allow drainage of any condensate that may form in the tube.

Examples of dry sprinklers are generally disclosed in U.S. Patents Nos. 5,775,431 to Ondracek and 5,967,240 to Ondracek. As shown generally in these patents, the actuating mechanism can be a rod or other similar structure that extends through the tube between the sprinkler head and the inlet end to maintain the seal at the inlet end. The actuating mechanism includes a thermally responsive support element at the sprinkler head that supports the rod and therefore the seal at the inlet end. In some sprinklers, the tube is also sealed at the sprinkler head end of the tube and the actuating mechanism is supported at the sprinkler head end by a seal cap supported by the thermally responsive support element. In such arrangements, the space in the tube between the two seal caps can be pressurized with a gas, such as dry air or nitrogen or with a liquid such as an antifreeze solution. When an elevated temperature is experienced, the thermally responsive support element fails releasing the plug seal (and also any lower seal at the sprinkler head end of the tube) to allow water from the supply conduit to flow into and through the tube to the sprinkler head, whereupon the fluid is distributed by the sprinkler head.

US 2003/075343 discloses a dry sprinkler comprises a tubular body having a connector and a sprinkler head. The connector is adapted to be connected to the water pipe network of the fire suppression system and the sprinkler head is adapted to discharge water or other liquid suppressant. The tubular body defines a flow passage between the connector and the sprinkler head. First and second seals are arranged in spaced relation along the flow passage and contain an antifreeze fluid therebetween. The antifreeze fluid may be a liquid or gas subjected to pressure. A thermally responsive element is arranged to maintain the second seal in a state that contains the antifreeze fluid between the first and second seals. The thermally responsive element releases the second seal when an elevated temperature condition exists.

The two-part form of claim 1 is based on this document.

US 6,488,097 discloses a central hub which includes a plate and a sleeve attached to the plate and adapted to receive a sprinkler head. The sleeve may be a telescoping sleeve. By providing a central hub with the plate and sleeve, the central hub provides increased stability and versatility to the sprinkler head.

Conventional dry sprinklers are fabricated using a rigid tube having a seal at the inlet that is separated from the sprinkler's temperature sensor, which is intended to be positioned in an area exposed to freezing conditions, such as an area that is not heated. The rigid tube extends into the unheated area from a wet pipe system (located in a heated area) and must be precisely aligned and installed while avoiding various architectural, structural and mechanical obstructions typically found in commercial or industrial buildings.

### SUMMARY

To remedy some of the problems and difficulties noted above, a dry sprinkler is provided as claimed as in claim 1. Preferred embodiments are disclosed in the dependent claims. The dry sprinkler has a flexible tube section that has openings at opposite ends . The sprinkler includes a supply line connection at one end of the tube section, the supply line connection having an opening to receive fluid from the supply line. The supply line connection is sealed with a first seal to prevent fluid from the supply from entering the tube section. A sprinkler head is coupled to the tube section at another end of the tube section opposite the supply line connection, the sprinkler head having a deflector, a second seal sealing an output orifice of the sprinkler head, and a thermally responsive element arranged to maintain the second seal sealed. A fluid that is not susceptible to freezing is contained in the tube between the first seal and the second seal. The thermally responsive element is constructed to release the second seal in response to an elevated temperature condition, to permit the release of the fluid in the tube and release the first seal to move toward the sprinkler head.

### BRIEF DESCRIPTION OF THE DRAWINGS

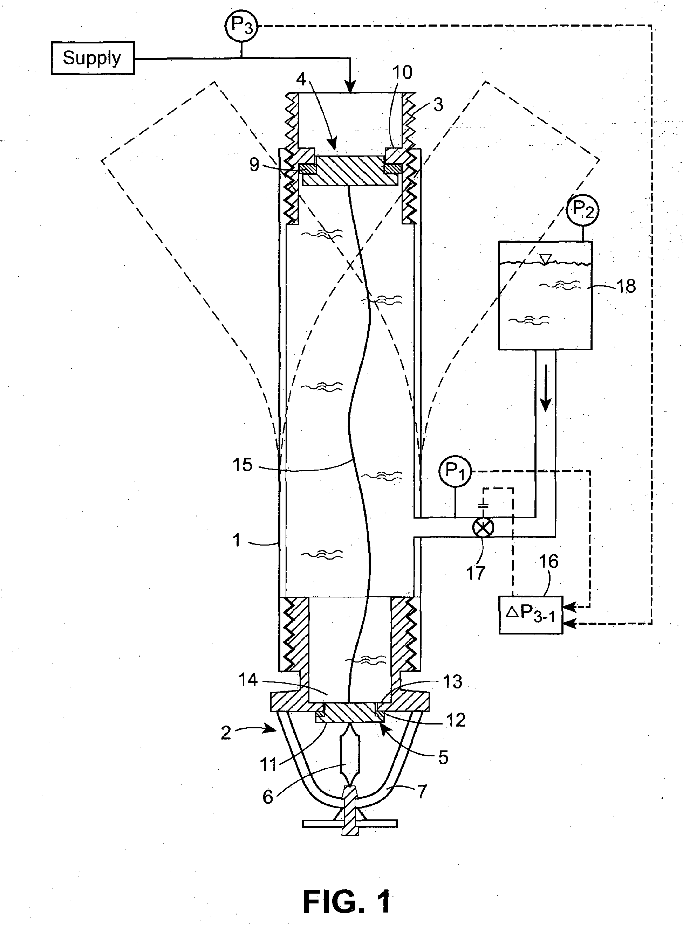

Fig. 1 shows a fire sprinkler system which includes a dry sprinkler in accordance with an embodiment of the invention.

### DETAILED DESCRIPTION

Figure 1 shows an embodiment of a flexible dry sprinkler arrangement comprised of a generally flexible tube 1, connected at one end to a sprinkler head 2 and connected at another end to a fitting 3 constructed to couple to a wet pipe, dry pipe, or preaction fire sprinkler system. The fitting 3 includes a first seal 4 that is normally in a closed or sealed position for preventing the flow of fluid from the fitting through the flexible tube and sprinkler head. The tube 1 is also sealed at the sprinkler head 2 by a second seal 5 (e.g., a sealing cap), which is supported by a thermal release element 6 compressed between the second seal and a frame 7 of the sprinkler head 2. The first seal 4 is generally formed as an annular plug having a grooved outer edge that seats against an annular spring washer 9, such as a Belleville washer. The spring washer 9 is configured to seal with an annular flange 10 extending from the inner wall of the fitting 3. The second seal 5 is formed similarly to the first seal 4, and also is generally formed as an annular disk 11 having an outer annular groove that receives a spring washer 12 which is constructed to seal against a sealing flange 13 formed at the output orifice 14 of the sprinkler head 2.

The tube 1 is flexible and can be formed from a metallic or non-metallic material. For example, in one embodiment, the tube is formed from a corrugated metal hose, and in another embodiment, the tube is formed from a corrugated plastic hose. The outer surface of the tube can be covered, such as with a braided jacket to protect the tube 1. As shown in a preferred embodiment, the sprinkler head 2 has male threads that engage with female threads on the output end of the tube 1. The female threaded connection of the tube 1 can include nominal diameter sizes of between 1.27 cm and 2.54 cm (i.e. ½ inch and 1 inch). While a specific configuration of sprinkler head 2 is shown in the figures, the present disclosure is broader than this configuration, and it is contemplated that substantially any present or future approved or listed fire sprinkler can be attached to the tube 1 in place of the sprinkler head 2 shown in Fig. 1.

In its sealed state shown in Fig. 1, the dry sprinkler is filled with a fluid and is pressurized to keep the first seal 4 sealed. The fluid is a freeze resistant gas such as nitrogen or a liquid such as a water/glycol mixture or other conventional "antifreeze" liquids.

The connection to the fire sprinkler system utilizes a differential pressure device 16 to ensure the pressure in the tube 1 is greater than the pressure in the fluid supply so as to prevent the introduction of water or air from the fire sprinkler system into the tube 1. This differential device is configured to maintain a ratio of 3 to 1 or larger between the pressure P1 in the tube 1 and the supply pressure P3. It is believed that the ratio of 3 to 1 is sufficient to prevent leakage into the tube 1 caused by pressure surges in the supply of fire sprinkler system. In one embodiment, the differential pressure device is configured as a differential pressure controller in communication with a pressure sensor monitoring the supply pressure P3 and is in communication with a pressure sensor monitoring the pressure P1. The differential pressure controller 16 is in communication with a control valve 17 positioned between the tube 1 and a source of pressurized fluid maintained at a pressure P2 higher than the fluid supply pressure P3. In the event that the pressure ratio drops below 3 to 1, a signal is sent from the controller 16 to the control valve 17 to open the valve 17 to introduce the high pressure fluid from supply 18 into the tube 1 until the 3 to 1 ratio is achieved, at which time the control valve 17 is instructed to close. Thus, the differential pressure controller 16 and control valve 17 operate according to a conventional feedback control arrangement to maintain the desired pressure ratio, e.g., of 3 to 1. It will be appreciated that the differential pressure controller may include a computer constructed to execute a computer readable program stored in a tangible computer-readable medium or in other memory, and including instructions for operating the controller to maintain the pressure ratio between P1 and P3. While one embodiment of a control arrangement has been described with reference to the preferred embodiment, it will be appreciated that other control arrangements other than that described herein can be employed to maintain the desired pressure ratio, and are within the scope of this disclosure.

Moreover, the various pressure sensors monitoring pressures P1, P2, and P3 can be constructed to communicate with an alarm system to notify an operator regarding operation of the sprinkler system. For example, an alarm may be provided for the pressure P1 to notify an operator if the pressure decreases to a certain limit, which may alert the operator of the possibility that the sprinkler head 2 may be leaking. It will be appreciated that notifications can be provided by various methods, including, but not limited to, a telephone message, e-mail, visual display, and a facsimile message. Optionally, a visual pressure indicator, such as an analog or digital pressure gauge, located proximate to the sprinkler, can be installed for indication that the fluid sealed in the tube has maintained its sealed pressure to within the tolerances permitted by its design. Such an indicator may simply be a binary status indicator such as a color, e.g., green, indicating acceptable operating status, and red, indicating an unacceptable operating status. Of course other pressure indicators are within the scope of this disclosure.

A flexible linkage 15 is shown in Fig. 1 connecting the first seal 4 to the second seal 5. The flexible linkage 15 is formed of a material that will not corrode in the presence of the fluid contained in the tube 1. The flexible linkage 15 can be formed as a chain or a cable. In one exemplary embodiment, the flexible linkage is constructed of stainless steel. By virtue of the flexible linkage 15, when the tube 1 of the sprinkler is bent as shown by the broken lines, the linkage 15 in the tube will conform to the inner wall of the tube 1 and not limit the range of motion of the tube 1. Also, physically coupling the first and second sealing caps together with the flexible linkage 15, will permit the first seal 4 to be pulled out of the tube by virtue of the release and discharge of the second seal 5. It is expected that the linkage 15 will positively ensure that the first seal 4 is indeed removed from the path of flow in the tube 1 without being obstructed by other structures in the tube 1. Also, the linkage 15 allows the first seal 4 to pass out of the tube 1 regardless of any kinks or sharp corners in the inner surface of the tube caused by corrugations in the tube 1 or flexing of the tube 1.

By virtue of the flexibility in the tube 1 of the sprinkler, installation of the sprinkler system is facilitated because the sprinkler can be moved around building obstructions that would ordinarily require additional plumbing work. Moreover, because the tube 1 is flexible, installers of the fluid supply need not have to precisely align the sprinkler drops in the ceiling of structures because any variance can be adjusted by movement of the tube 1.

While the present invention has been described with respect to what is presently considered to be the preferred embodiments, it is to be understood that the invention is not limited to the disclosed embodiments. To the contrary, the invention is intended to cover various modifications and equivalent arrangements included within the scope of the appended claims.

## Claims

1. A dry sprinkler comprising:
a tube section (1) having openings at opposite ends of the tube section;
a supply line connection (3) at one end of the tube section (1), the supply line connection (3) having an opening to receive fluid from the supply line;
a first seal (4) sealed with the supply line connection to prevent fluid from the supply from entering the tube section;
a sprinkler head (2) coupled to the tube section (1) at another end of the tube section opposite the supply line connection, the sprinkler head (2) having a deflector, a second seal (5) sealing an output orifice of the sprinkler head, and a thermally responsive element (6) arranged to maintain the second seal (5) sealed; and
a fluid contained in the tube between the first seal (4) and the second seal (5), wherein the fluid is not susceptible to freezing,
wherein the thermally responsive element (6) is constructed to release the second seal (5) in response to an elevated temperature condition, to permit the release of the fluid in the tube and release the first seal (4) to move toward the sprinkler head (2),
**characterized in that** the tube section (1) is flexible and the first seal (4) and the second seal (5) are connected by a flexible connector (15); and
the dry sprinkler further comprises a differential pressure device (16) constructed to maintain a ratio between the pressure in the tube section (1) and the pressure of the fluid supply to at least a certain ratio.

2. The dry sprinkler according to claim 1, wherein the fluid includes one of a gas and a liquid.

3. The dry sprinkler according to claim 2, wherein the fluid includes a liquid antifreeze solution.

4. The dry sprinkler according to claim 1, wherein the differential pressure device (16) is constructed to maintain the ratio between the pressure in the tube section (1) and the pressure of the fluid supply to at least 3 to 1.

5. The dry sprinkler according to any preceding claim, further comprising a threaded connection joining the tube section (1) to the sprinkler head (2).

6. The dry sprinkler according to any preceding claim, further comprising a pressure indicator coupled to the tube section (1), the pressure indicator constructed to communicate the pressure of the fluid in the tube section (1).

7. The dry sprinkler according to claim 6, wherein the pressure indicator is at least one of an analog and a digital pressure monitor.

8. The dry sprinkler according to claim 7, wherein the indicator is configured to transmit a signal to a pressure monitoring device.

9. The dry sprinkler according to claim 1, wherein the flexible connector (15) includes at least one of a chain and a cable.

10. The dry sprinkler according to any preceding claim, wherein the inner surface of the tube section (1) has a circular cross-section, and the first seal (4) has a circular periphery and an annular groove constructed to receive a spring washer (9) having a diameter smaller than the inner surface of the tube section (1).

11. A dry sprinkler according to claim 10, wherein the supply line connection includes an annular flange (10) extending from the inner wall of the supply line connection which is constructed to seal with the spring washer (9).

## Patentansprüche

1. Trockensprinkler, aufweisend:
einen Kanalabschnitt (1) mit Öffnungen an gegenüberliegenden Enden des Kanalabschnitts;
einen Zufuhr-Leitungsanschluss (3) an einem Ende des Kanalabschnitts (1), wobei der Zufuhrleitungsabschnitt (3) eine Öffnung hat, um ein Fluid von der Zufuhrleitung zu empfangen;
eine erste Dichtung (4) in Dichtungsabschluss mit dem Zufuhrleitungsanschluss, um zu verhindern, dass Fluid von der Zufuhr in den Kanalabschnitt eindringt;
einen Sprinklerkopf (2), der an den Kanalabschnitt an einem anderen Ende des Kanalabschnitts gegenüberliegend dem Zufuhrleitungsanschluss gekoppelt ist, wobei der Sprinklerkopf (2) einen Deflektor aufweist, eine zweite Dichtung (5), die eine Ausgangsöffnung des Sprinklerkopfs abdichtet, sowie ein thermisches Rückkopplungselement (6), dass dazu angeordnet ist, die zweite Dichtung (5) in Dichtfunktion zu halten; und
ein Fluid, das in dem Kanal zwischen der ersten Dichtung (4) und der zweiten Dichtung (5) enthalten ist, wobei das Fluid frostsicher ist,
wobei das thermische Rückkopplungselement (6) konstruiert ist, um die zweite Dichtung (5) in Antwort auf einen erhöhten Temperaturzustand freizugeben, um die Freigabe des Fluides in dem Kanal zu ermöglichen, und die erste Dichtung (4) für eine Bewegung in Richtung zu dem Sprinklerkopf (2) freizugeben,
**dadurch gekennzeichnet, dass** der Kanalabschnitt (1) flexibel ist und die erste Dichtung (4) und die zweite Dichtung (5) durch einen flexiblen Anschluss (15) angeschlossen sind; und der Trockensprinkler ferner eine Differenzial-Druckvorrichtung (16) aufweist, die konstruiert ist, um ein Verhältnis zwischen dem Druck in dem Kanalabschnitt (1) und dem Druck der Fluidzufuhr auf zumindest ein bestimmtes Verhältnis aufrecht zu erhalten.

2. Trockensprinkler nach Anspruch 1, wobei das Fluid entweder ein Gas oder eine Flüssigkeit enthält.

3. Trockensprinkler nach Anspruch 2, wobei das Fluid eine flüssige Frostschutzlösung enthält.

4. Trockensprinkler nach Anspruch 1, wobei die Differenzial-Druckvorrichtung (16) konstruiert ist, um das Verhältnis zwischen dem Druck in dem Kanalabschnitt (1) und dem Druck der Fluidzufuhr auf zumindest 3:1 zu halten.

5. Trockensprinkler nach einem der vorangehenden Ansprüche, ferner aufweisend einen Schraubgewinde-Anschluss, der den Kanalabschnitt (1) mit dem Sprinklerkopf (2) verbindet.

6. Trockensprinkler nach einem der vorangehenden Ansprüche, ferner aufweisend einen Druckindikator, der an dem Kanalabschnitt (1) angekoppelt ist, wobei der Druckindikator dazu konstruiert ist, um den Druck des Fluides in dem Kanalabschnitt (1) zu übbermitteln.

7. Trockensprinkler nach Anspruch 6, wobei der Druckindikator entweder ein analoger oder ein digitaler Druckmonitor ist.

8. Trockensprinkler nach Anspruch 7, wobei der Indikator dazu konfiguriert ist, ein Signal an eine Druck-Monitorvorrichtung zu übertragen.

9. Trockensprinkler nach Anspruch 1, wobei der flexible Anschluss (15) zumindest ein Zugorgan oder ein Kabel aufweist.

10. Trockensprinkler nach einem der vorangehenden Ansprüche, wobei die Innenfläche des Kanalabschnitts (1) einen kreisförmigen Querschnitt hat, und die erste Dichtung (4) einen kreisförmigen Außenumfang hat, sowie eine ringförmige Nut, die dazu konstruiert ist, einen Federring (9) aufzunehmen, mit einem Durchmesser, der kleiner ist als die Innenfläche des Kanalabschnitts (1).

11. Trockensprinkler nach Anspruch 10, wobei der Zufuhrleitungsanschluss einen ringförmigen Flansch (10) aufweist, der sich von der Innenwand des Zufuhrleitungsanschlusses erstreckt, der dazu konstruiert ist, mit dem Federring (9) abzudichten.

## Revendications

1. Extincteur automatique sans air qui comprend :
une section de tube (1) qui présente des ouvertures aux extrémités opposées de la section de tube ;
un raccord de conduite d'alimentation (3) à une extrémité de la section de tube (1), le raccord de conduite d'alimentation (3) ayant une ouverture destinée à recevoir un fluide de la part de la conduite d'alimentation ;
un premier joint (4) fermé avec le raccord de conduite d'alimentation afin d'empêcher le fluide qui provient de l'alimentation de pénétrer dans la section de tube ;
une tête d'extincteur automatique (2) reliée à la section de tube (1) à une autre extrémité de la section de tube opposée au raccord de conduite d'alimentation, la tête d'extincteur automatique (2) ayant un déflecteur, un second joint (5) qui ferme un orifice de sortie de la tête d'extincteur automatique, et un élément thermiquement réactif (6) placé afin de maintenir le second joint (5) fermé ; et
un fluide contenu dans le tube entre le premier joint (4) et le second joint (5), le fluide n'étant pas susceptible de geler,
dans lequel l'élément thermiquement réactif (6) est construit de façon à libérer le second joint (5) en réponse à une condition d'élévation de température, afin de permettre la libération du fluide dans le tube, et à libérer le premier joint (4) afin qu'il se déplace vers la tête d'extincteur automatique (2),
**caractérisé en ce que** la section de tube (1) est flexible et le premier joint (4) et le second joint (5) sont reliés par un raccord flexible (15) ; et
l'extincteur automatique sans air comprenant en outre un dispositif à pression différentielle (16) construit de façon à maintenir au moins un certain rapport entre la pression dans la section de tube (1) et la pression de l'alimentation en fluide .

2. Extincteur automatique sans air selon la revendication 1, dans lequel le fluide comprend l'un d'un gaz et d'un liquide.

3. Extincteur automatique sans air selon la revendication 2, dans lequel le fluide comprend une solution antigel liquide.

4. Extincteur automatique sans air selon la revendication 1, dans lequel le dispositif à pression différentielle (16) est construit de façon à maintenir le rapport entre la pression dans la section de tube (1) et la pression de l'alimentation en fluide à au moins 3:1.

5. Extincteur automatique sans air selon l'une quelconque des revendications précédentes, qui comprend en outre un raccord fileté qui joint la section de tube (1) à la tête d'extincteur automatique (2).

6. Extincteur automatique sans air selon l'une quelconque des revendications précédentes, qui comprend en outre un indicateur de pression relié à la section de tube (1), l'indicateur de pression étant construit de façon à communiquer la pression du fluide dans le tube (1).

7. Extincteur automatique sans air selon la revendication 6, dans lequel l'indicateur de pression est au moins l'un d'un moniteur de pression analogique et d'un moniteur de pression numérique.

8. Extincteur automatique sans air selon la revendication 7, dans lequel l'indicateur est configuré pour transmettre un signal à un dispositif de surveillance de la pression.

9. Extincteur automatique sans air selon la revendication 1, dans lequel le raccord flexible (15) comprend au moins l'une d'une chaîne et d'un câble.

10. Extincteur automatique sans air selon l'une quelconque des revendications précédentes, dans lequel la surface intérieure de la section de tube (1) possède une section transversale, et le premier joint (4) possède une périphérie circulaire et une rainure annulaire construite de façon à recevoir une rondelle élastique (9) qui présente un diamètre inférieur à la surface intérieure de la section de tube (1).

11. Extincteur automatique sans air selon la revendication 10, dans lequel le raccord de conduite d'alimentation comprend une bride annulaire (10) qui s'étend depuis la paroi intérieure du raccord de conduite d'alimentation qui est construite de façon à se sceller avec la rondelle élastique (9).
